# EUROPÄISCHE PATENTSCHRIFT

(11) **EP 1 320 527 B1**
(45) Veröffentlichungstag und Bekanntmachung des Hinweises auf die Patenterteilung: **13.08.2008**
(21) Anmeldenummer: 01983513.1
(22) Anmeldetag: 28.09.2001
(51) Int. Cl.: C07D 231/16, C07D 231/14, C07D 231/20

(54) **VERFAHREN ZUR HERSTELLUNG VON 1-SUBSTITUIERTEN 5-CHLOR-4METHYLPYRAZOLEN**
METHOD FOR PRODUCING 1 SUBSTITUTED 5-CHLORO-4 METHYL PYRAZOLES
PROCEDE DE PRODUCTION DE 5-CHLORE-4-METHYLPYRAZOLES SUBSTITUES EN POSITION 1

(30) Priorität: 29.09.2000 DE 10048384
(43) Veröffentlichungstag der Anmeldung: 25.06.2003
(73) Patentinhaber: BASF SE, 67056 Ludwigshafen (DE)
(72) Erfinder: MERKLE, Hans, Rupert, 67061 Ludwigshafen (DE); FRETSCHNER, Erich, 69239 Neckarsteinach (DE)
(74) Vertreter: Pohl, Michael Friedrich
(86) Internationale Anmeldenummer: PCT/EP2001/011259
(87) Internationale Veröffentlichungsnummer: WO 2002/026715

(56) Entgegenhaltungen:
- EP-A- 0 366 329
- CHEMICAL ABSTRACTS, vol. 66, no. 21, 22. Mai 1967 (1967-05-22) Columbus, Ohio, US; abstract no. 94950x, J. ELGUERO ET AL.: "Azoles. Bromination of pyrazolecarboxylic acids and esters" Seite 8889; Spalte 1; XP002188366 & C.R.ACAD.SCI., Bd. 263, Nr. 23, - 1966 Seiten 1456-1459, Paris

## Beschreibung

Die vorliegende Erfindung betrifft ein Verfahren zur Herstellung N-substituierter 5-Chlor-4-methylpyrazole der allgemeinen Formel I, 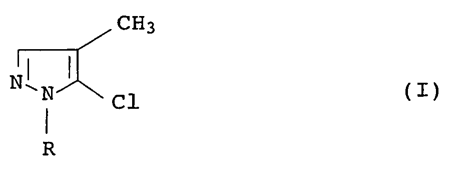 worin
- R: für C₁-C₈-Alkyl oder C₅-C₁₀-Cycloalkyl steht, die gegebenen-falls einen oder mehrere Substituenten aufweisen.

1-Alkyl-4-methyl-5-chlorpyrazole sind wichtige Ausgangsstoffe für die Herstellung von Pharmazeutika und Pflanzenschutzmitteln.

Die EP 0366 329 A1 beschreibt die Herstellung von 5-Halogen-4-methylpyrazolen und 3,5-Dihalogen-4-methylpyrazolen durch Umsetzung von 4-Methylpyrazolen mit Halogen.

Das in EP 0366 329 A1 beschriebene Verfahren weist den Nachteil auf, dass bei der Chlorierung ein Gemisch aus mono- und dichlorierten Verbindungen entsteht. Hierdurch geht ein Teil des wertvollen Ausgangsmaterials in Form des dichlorierten Pyrazols verloren, und die Ausbeute an 5-Chlor-4-methylpyrazol I bezogen auf die eingesetzte 4-Methylpyrazolverbindung II ist nur mäßig. Der vorliegenden Erfindung liegt somit die Aufgabe zugrunde, ein wirtschaftliches Verfahren zur Herstellung von 5-Chlor-4-methylpyrazolen der Formel I bereitzustellen, das die Zielverbindung in besseren Ausbeuten, bezogen auf die als Ausgangsverbindung eingesetzte 4-Methylpyrazolverbindung liefert.

Diese Aufgabe wurde gelöst durch ein Verfahren, bei dem man zunächst eine 4-Methylpyrazolverbindung mit Chlor umsetzt, das Reaktionsprodukt in das Mono- und Dichlorpyrazol auftrennt, das Dichlorpyrazol enthalogeniert und in die Umsetzung mit Chlor zurückführt.

Demnach betrifft die vorliegende Erfindung ein Verfahren zur Herstellung von 1-substituierten 5-Chlor-4-methylpyrazolen der Formel I, durch Umsetzung einer 4-Methylpyrazolverbindung der allgemeinen Formel II, 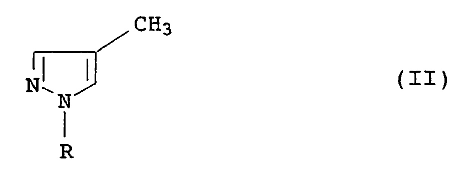 worin R die oben genannten Bedeutungen aufweist, mit Chlor, wobei man ein Gemisch aus Verbindung I und einer 1-substituierten 3,5-Dichlor-4-methylpyrazol-Verbindung der Formel III erhält, 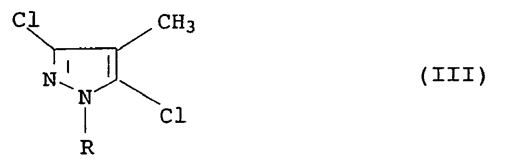 worin R die zuvor genannten Bedeutungen hat, dadurch gekennzeichnet, dass man die Verbindung III von der Verbindung I abtrennt, die Verbindung III zu der Verbindung II enthalogeniert und diese erneut mit Chlor umsetzt.

Die nach dem erfindungsgemäßen Verfahren in einer hohen Gesamtausbeute erhältlichen 5-Chlor-4-methylpyrazole I können außerdem in N-substituierte 2-Pyrazolin-5-one umgewandelt werden, die ebenfalls wertvolle Zwischenprodukte in der Herstellung von Pharmazeutika und Pflanzenschutzmitteln darstellen. Daher ist ein weiterer Aspekt der vorliegenden Erfindung die Bereitstellung eines Verfahrens zur Herstellung von N-substituierten 2-Pyrazolin-5-onen ausgehend von 1-substituierten 5-Chlor-4-methylpyrazolen der Formel I.

In der vorliegenden Erfindung ist die Art des Substituenten R von untergeordneter Bedeutung. So bedeuten:
C₁-C₈-Alkyl: eine lineare oder verzweigte Alkylkette mit 1 bis 8 C-Atomen, z. B. Methyl, Ethyl, n-Propyl, iso-Propyl, n-Butyl, 2-Butyl, iso-Butyl, tert.-Butyl, n-Pentyl, 2-Methylbutyl, 2,2-Dimethylpropyl, n-Hexyl, 1-Methylpentyl, 2-Methylpentyl, 1-Ethylbutyl, 2-Ethylbutyl, n-Heptyl, n-Octyl und 2-Ethylhexyl.
C₅-C₁₀-Cycloalkyl: mono- oder bicyclische Kohlenwasserstoffreste mit 5 bis 10 Kohlenstoffatomen, z. B. Cyclopentyl, Cyclohexyl, Cycloheptyl, Cyclooctyl, Cyclononyl, Cyclodecyl, Norbornyl, Bicyclo[2.2.2]octyl und Dekahydronaphthyl.

Die vorgenannten Reste können einen oder mehrere Substituenten aufweisen. Beispiele für derartige Reste sind Halogen wie Fluor oder Chlor, Halogenalkyl wie Trifluormethyl, Pentafluorethyl sowie Fluoralkoxy wie Trifluormethoxy und Pentafluorethoxy. Darüber hinaus kommt für den Rest Cycloalkyl in Betracht.

Die Ausgangsverbindungen der Formel II sind dem Fachmann bekannt und zugänglich (siehe z. B. EP 0 366 329 A1 und dort zitierte Literatur).

Die Umsetzung der 4-Methylpyrazole II mit Chlor erfolgt nach den für eine Chlorierung von Pyrazolen üblichen Methoden, z. B. nach der in der EP 366 329 A1 beschriebenen Methode, auf die hiermit Bezug genommen wird. Vorzugsweise führt man die Chlorierung in einem inerten organischen Lösungsmittel durch. Als Lösungsmittel verwendet man z. B. halogenierte, aliphatische Kohlenwasserstoffe, wie 1,2-Dichlorethan, Dichlormethan, Dichlorpropan, 1-Chlorpentan.

Die Reaktionstemperatur liegt in der Regel zwischen Raumtemperatur und Siedetemperatur des Lösungsmittels und wird im Bereich von etwa 40 °C bis etwa 70 °C gehalten.

Die Umsetzung von Verbindung II mit Chlor erfolgt in der Regel so, dass man in ein Reaktionsgefäß, welches die Verbindung II enthält, bei der erforderlichen Reaktionstemperatur eine ausreichende Menge Chlor zugibt. Die Zugabe kann sowohl in Form einer chlorhaltigen Lösung, vorzugsweise in einem der vorgenannten Lösungsmittel, als auch durch Einleiten von Chlorgas erfolgen. Meistens setzt man Chlor bezogen auf das Pyrazol II im Hinblick auf eine vollständige Umsetzung im Überschuss ein. Vorzugsweise beträgt dieser bis 70 Mol%, insbesondere 10-60 Mol%. Ein erhöhter Anteil an Dichlorverbindung ist nicht weiter störend, da im erfindungsgemäßen Verfahren das Dichlorpyrazol enthalogeniert und in die Umsetzung mit Chlor zurückgeführt wird.

Das bei der Chlorierung anfallende Reaktionsgemisch wird in üblicher Weise aufgearbeitet und das dabei in einer Ausbeute > 95 %, bezogen auf die eingesetzte Verbindung II, anfallende Gemisch von Monochlorverbindung I und Dichlorverbindung III aufgetrennt, z. B. durch fraktionierte Destillation, vorzugsweise bei vermindertem Druck. Die Verbindung I fällt dabei in Reinform an, die direkt weiterverarbeitet werden kann. Erfindungsgemäß wird dann die Verbindung III, gegebenenfalls im Gemisch mit Verbindung I, zu Verbindung II enthalogeniert.

Die Enthalogenierung von III, bzw. einer Mischung der Verbindungen I und III, erfolgt nach den hierfür üblichen Verfahren. Eine Übersicht über verschiedene Verfahren zur Enthalogenierung findet man in Chem. Technik 6 (1994) 316-323 und der dort zitierten Literatur.

Die Dehalogenierung einer Verbindung III erfolgt vorzugsweise durch katalytische Hydrogenolyse. Der Partialdruck des Wasserstoffs liegt im Bereich von etwa 1 bar bis etwa 80 bar, besonders im Bereich von etwa 10 bar bis etwa 80 bar, insbesondere im Bereich von etwa 10 bar bis etwa 50 bar. In der Regel erfolgt die Dechlorierung bei erhöhter Temperatur, vorzugsweise zwischen etwa Raumtemperatur und etwa 150 °C, insbesondere zwischen etwa 50 °C und etwa 100 °C. Die Reaktionsdauer hängt erwartungsgemäß von den gewählten Reaktionsbedingungen sowie von der verwendeten Verbindung III ab.

Als Katalysatoren für die Hydrogenolyse wird man in der Regel Übergangsmetalle und deren Verbindungen oder Komplexe einsetzen, wobei die Katalysatoren vorzugsweise in geträgerter Form eingesetzt werden. Als Übergangsmetalle sind insbesondere die Metalle der VIII. Nebengruppe und ganz besonders die Platinmetalle wie Palladium, Rhodium und Platin bevorzugt.

Geeignete Trägermaterialien umfassen sowohl anorganische Träger wie Titandioxid, Kieselgel, Kieselsäure, Zeolithe, Aluminiumoxid als auch organische Polymere oder Aktivkohle. In einer bevorzugten Ausführungsform der Erfindung verwendet man als Katalysator Palladium auf Aktivkohle.

Zum Abfangen des gebildeten Chlorwasserstoffs führt man die Hydrogenolyse vorzugsweise in Gegenwart einer geeigneten Base, wie einem tert. Amin, beispielsweise Triethylamin oder einem basischen Salz, wie ein Alkaliacetat oder Erdalkaliacetat, insbesondere Natriumacetat, oder wie ein Alkalicarbonat oder Alkalihydrogencarbonat, wie Natriumcarbonat oder Natriumhydrogencarbonat durch. Geeignete Basen sind ferner Alkalihydroxide, wie Natriumhydroxid oder Kaliumhydroxid und Erdalkalihydroxide wie Calciumhydroxid oder Magnesiumhydroxid. Ferner eignen sich Erdalkalioxide wie Calciumoxid oder Magnesiumoxid. Vorzugsweise setzt man wenigstens 2 Mol Base pro Mol der Verbindung III ein, da zwei Mol Chlorwasserstoff zu neutralisieren sind.

Die Dehalogenierung wird vorzugsweise in einem organischen Lösungsmittel durchgeführt. Besonders eignen sich die Ausgangsverbindung II, aliphatische C₁-C₈-Carbonsäuren wie Ameisensäure, Essigsäure, Propansäure, Pivalinsäure, Buttersäure und deren Gemische, insbesondere Essigsäure oder unter den Reaktionsbedingungen beständige Lösungsmittel wie Ether, beispielsweise Tetrahydrofuran, Dioxan, Carbonsäureester wie Essigester, aromatische Kohlenwasserstoffe wie Toluol oder aliphatische Kohlenwasserstoffe. In einer bevorzugten Ausführungsform verwendet man Eisessig und/oder die Ausgangsverbindung II als Lösungsmittel.

Die Aufarbeitung der bei der Dehalogenierung erhaltenen Reaktionsmischung nach herkömmlichen Verfahren ergibt das 4-Methylpyrazol der allgemeinen Formel II, das dann erneut der Chlorierung unterzogen wird.

Das erfindungsgemäße Verfahren ermöglicht somit die Überführung der Pyrazole der allgemeinen Formel II in die 5-Chlor-4-methylpyrazole der allgemeinen Formel I in hohen Ausbeuten.

Die nach dem erfindungsgemäßen Verfahren erhaltenen 5-Chlor-4-methylpyrazole der Formel I sind insbesondere im Hinblick auf die Synthese von N-substituierten 2-Pyrazolin-5-onen der allgemeinen Formel IV von Interesse, 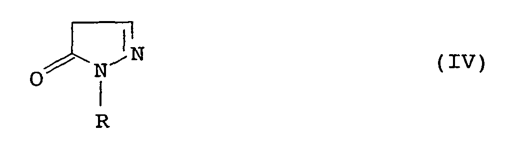 worin R wie vorstehend definiert ist. Es wurde nämlich von der Anmelderin gefunden, dass die 4-Methylgruppe in den Verbindungen der Formel I abgebaut werden kann und gleichzeitig die 5-Chlorfunktionalität in eine Hydroxygruppe umgewandelt werden kann. Das hierbei erhaltene 5-Hydroxypyrazol ist zu IV Tautomer und lagert sich dementsprechend in die Verbindung IV um bzw. steht mit ihr im Gleichgewicht.

Die Umwandlung des 5-Chlor-4-methylpyrazols erfolgt erfindungsgemäß dadurch, dass man die Methylgruppe der 4-Position zur Carboxylgruppe oxidiert und das hierbei erhaltene 4-Carboxy-5-chlorpyrazol der Formel V, worin R die zuvor genannten Bedeutungen aufweist, in einem wässrigen Reaktionsmedium bei erhöhter Temperatur mit einem molaren Überschuss Alkalihydroxid umsetzt und anschließend durch Zugabe einer Säure einen pH-Wert ≤ 6 im wässrigen Reaktionsmedium einstellt.

Verfahren zur Oxidation von aromatischen Methylgruppen zu Carboxylgruppen sind aus dem Stand der Technik bekannt, beispielsweise aus EP 224 094, US-A 3,801,584 und EP 350 176 A.

N-substituierte 5-Halogen-4-methylpyrazole werden vorzugsweise nach dem in EP-A 350 176 beschriebenen Verfahren in einfacher Weise zu den Carbonsäuren der Formel V oxidiert.

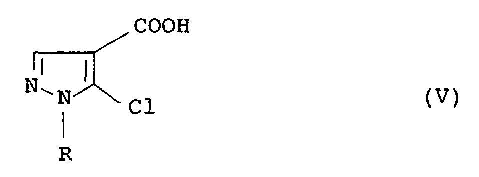

Die Oxidation erfolgt vorzugsweise mit Wasserstoffperoxid und/oder Sauerstoff. Als Sauerstoffquelle verwendet man reinen Sauerstoff oder Luft, wobei der Partialdruck des Sauerstoff-enthaltenden Gases in der Regel etwa 1 bis 93 bar beträgt. Vorzugsweise erfolgte die Oxidation durch Umsetzung von I mit Luftsauerstoff in Gegenwart einer Übergangsmetallverbindung bzw. eines Übergangsmetallsalzes, worin das Übergangsmetall in einer Oxidationsstufe > 0 vorliegt.

Geeignete Übergangsmetallsalze sind Salze des Mangans, Kobalts, Eisens und deren Mischungen wie Eisenformiat, Eisenacetat, Eisenlaktat, Eisenoxalat, Eisenoktylat, Eisenacetylacetonat, Eisenchlorid, Eisenbromid, Eiseniodid, Kobaltformiat, Kobaltacetat, Kobaltoktylat, Kobaltacetylacetonat, Kobaltiodid, Kobaltcarbonat, Manganformiat, Manganacetat, Manganoktylat, Manganacetylacetonat, Manganchlorid, Manganbromid, Manganiodid und Mangancarbonat.

Vorzugsweise führt man die Oxidation in Gegenwart von Bromidionen, z. B. in Form eines Erdalkali- oder Alkalibromids wie Natriumbromid, Kaliumbromid oder Ammoniumbromid durch.

Als Lösungsmittel verwendet man üblicherweise eine niedere Carbonsäure wie Essigsäure, Propionsäure, Buttersäure oder ein niederes Carbonsäureanhydrid wie Essigsäureanhydrid oder Propionsäureanhydrid. Die Reaktionstemperatur liegt in der Regel im Bereich von etwa 20 bis etwa 200 °C.

Zur Umwandlung von V in das Pyrazolon IV setzt man im erfindungsgemäßen Verfahren in einem ersten Schritt eine Verbindung der allgemeinen Formel V mit Alkalihydroxid in molarem Überschuss in einem wässrigen Reaktionsmedium um. Ein molarer Überschuss an Alkalihydroxid ist bei den Verbindungen der allgemeinen Formel V dann gewährleistet, wenn man pro Mol Verbindung V mehr als 2 Mol Alkalihydroxid einsetzt. In der ersten Stufe werden ein Mol für die Substitution von Cl durch Hydroxy und ein Mol für die Neutralisation der Carbonsäure benötigt. Erfindungsgemäß bevorzugt setzt man 3 bis 20 Mol Alkalihydroxid und insbesondere 5 bis 12 Mol Alkalihydroxid je Mol Verbindung V ein. Bevorzugte Alkalihydroxide sind Natriumhydroxid und Kaliumhydroxid, insbesondere Natriumhydroxid.

Als wässrige Reaktionsmedien kommen sowohl Wasser als auch Gemische aus Wasser und wassermischbaren organischen Lösungsmitteln in Betracht. Die wassermischbaren organischen Lösungsmittel sind vorzugsweise gegenüber Alkalihydroxid unter Reaktionsbedingungen inert. Beispiele für geeignete organische Lösungsmittel sind C₁-C₄-Alkanole, insbesondere Methanol und Ethanol, weiterhin Dimethylsulfoxid, Tetrahydrofuran, Dioxan, Glykol, Glycerin, Diethylenglykol, Triethylenglykol und vergleichbare. In der Regel wird das wässrige Reaktionsmedium nicht mehr als 50 Vol-%, vorzugsweise nicht mehr als 30 Vol-% und insbesondere nicht mehr als 10 Vol-% eines organischen, wassermischbaren Lösungsmittels enthalten. In einer bevorzugten Ausführungsform der vorliegenden Erfindung ist Wasser alleiniges Lösungsmittel.

Besonders bevorzugt führt man den ersten Reaktionsschritt in einer wässrigen Alkalihydroxid-Lösung durch, die 10 bis 50 Gew.-% und insbesondere 20 bis 40 Gew.-% Alkalihydroxid enthält.

Erfindungsgemäß wird der erste Reaktionsschritt bei erhöhter Temperatur durchgeführt. Unter erhöhter Temperatur versteht man ein Erwärmen auf in der Regel wenigstens 50 °C und vorzugsweise wenigstens 90 °C. In der Regel wird die Reaktionstemperatur 200 °C nicht überschreiten. Ganz besonders bevorzugt führt man die Reaktion bei Temperaturen im Bereich von 120 bis 200 °C durch.

Je nach Reaktionstemperatur wird man den ersten Reaktionsschritt drucklos oder unter erhöhtem Druck durchführen. Bei Reaktionstemperaturen oberhalb 100 °C stellt sich in der Regel ein Reaktionsdruck von 1 bis 10 bar ein. Typische Reaktionsbedingungen sind beispielsweise bei einem rein wässrigen Reaktionsmedium 150 bis 180 °C und 5 bis 7 bar.

In der Regel wird man die Reaktion zu einem nahezu vollständigen Umsatz der Ausgangsverbindung V führen. Unter Umsatz versteht man hier die Umwandlung von Cl im Pyrazol V in eine Hydroxylgruppe bzw. die Bildung des entsprechenden Alkoholats. Die Dauer zur Erreichung eines nahezu vollständigen Umsatzes hängt naturgemäß von den gewählten Reaktionsbedingungen ab und kann zwischen 0,5 h und 24 h variieren. Typische Reaktionsdauern in rein wässrigen Systemen liegen in der Regel im Bereich von 2 bis 10 h.

Im zweiten Reaktionsschritt setzt man das im ersten Reaktionsschritt erhaltene Reaktionsprodukt unter sauren Bedingungen um. Hierbei tritt unter CO₂-Entwicklung die Bildung der Verbindung IV ein. Die CO₂-Entwicklung ist auf die Abspaltung der in der 4-Position des Pyrazolrings befindlichen Carboxylgruppe zurückzuführen.

Die Durchführung der zweiten Reaktionsstufe erfolgt in der Regel ohne Isolierung des in der ersten Reaktionsstufe gebildeten Reaktionsproduktes. Vorzugsweise leitet man die zweite Reaktionsstufe durch Zugabe einer Säure in die Reaktionsmischung der ersten Reaktionsstufe ein. Gegebenenfalls kann man auch das wässrige Lösungsmittel der ersten Reaktionsstufe vor der Durchführung der zweiten Reaktionsstufe teilweise oder vollständig entfernen und durch ein neues Lösungsmittel, vorzugsweise ein wässriges Lösungsmittel und insbesondere durch Wasser ersetzen. Diese Vorgehensweise bietet sich insbesondere dann an, wenn in der ersten Stufe ein organisches Lösungsmittel eingesetzt wurde, das beispielsweise aufgrund einer mit der Verbindung IV vergleichbaren Flüchtigkeit oder in sonstiger Weise deren Isolierung erschwert.

Erfindungsgemäß wird die zweite Reaktionsstufe unter sauren Bedingungen durchgeführt, d. h. der pH-Wert der Reaktionsmischung in der zweiten Reaktionsstufe beträgt höchstens 6 und liegt vorzugsweise im Bereich von 1 bis 3. Ein pH-Wert von 0 wird vorzugsweise nicht unterschritten. Die Einstellung des pH-Werts erfolgt durch Zugabe einer Säure zu dem Reaktionsprodukt der ersten Reaktionsstufe. Vorzugsweise gibt man die Säure zu der wässrigen Reaktionsmischung der ersten Reaktionsstufe. In der Regel wird man so vorgehen, dass man die Reaktionsmischung der ersten Reaktionsstufe auf eine für die zweite Reaktionsstufe geeignete Temperatur abkühlt, die in der Regel im Bereich von etwa 0 bis 100 °C und vorzugsweise im Bereich von etwa 10 bis 50 °C liegt und dann die Säure zugibt.

Als Säuren kommen grundsätzlich alle Säuren in Betracht, die eine zur Erreichung des gewünschten pH-werts ausreichende Säurestärke besitzen. Sofern man die zweite Reaktionsstufe direkt im Anschluss an die erste Reaktionsstufe durchführt, muss berücksichtigt werden, dass überschüssiges Alkalihydroxid noch neutralisiert werden muss. Aus diesem Grund wird man zur Einstellung des pH-Werts eine starke Säure, vorzugsweise eine Mineralsäure, wie Salzsäure, Schwefelsäure oder Phosphorsäure, einsetzen. Vorzugsweise werden die Säuren und insbesondere Salzsäure, Phosphorsäure und Schwefelsäure in einer verdünnten wässrigen Form eingesetzt.

Sofern die erste Reaktionsstufe unter Druck durchgeführt wurde, empfiehlt es sich, vor der Neutralisation mit der Säure den Reaktor zu entspannen. In der Regel setzt die Decarboxylierung spontan bei Zugabe der Säure ein, wenn der geeignete pH-Wert erreicht ist. Gewünschtenfalls kann man zur Vervollständigung der Decarboxylierung die Reaktionsbedingungen noch einen gewissen Zeitraum, der wenige Minuten bis einige Stunden betragen kann, beibehalten. Die Isolierung der Verbindung IV erfolgt in üblicher Weise durch Aufarbeitung der Reaktionsmischungen des zweiten Reaktionsschrittes nach üblichen Aufarbeitungsmethoden, beispielsweise durch extraktive Aufarbeitung der flüssigen Reaktionsmischung mit einem organischen Lösungsmittel oder durch Entfernen des Lösungsmittels und Isolieren der Zielverbindung aus dem dabei erhaltenen Rückstand. Vor der Aufarbeitung empfiehlt es sich, die Reaktionsmischung der zweiten Reaktionsstufe mit einer Base auf pH-Werte ≥ 6, z.B. pH 6 bis 7, zu neutralisieren. Geeignete Basen sind Alkalihydroxide, Alkalicarbonate, Alkalihydrogencarbonate, Erdalkalicarbonate und Erdalkalihydroxide. Üblicherweise wird man Alkalihydroxide und insbesondere Natronlauge zur Neutralisation einsetzen.

Im erfindungsgemäßen Verfahren ist es aufgrund der anfallenden Salzfracht häufig vorteilhaft, zur Isolierung der Verbindung IV das wässrige Reaktionsmedium der 2. Reaktionsstufe, vorzugsweise nach Neutralisation, destillativ oder durch Verdampfen im Vakuum weitgehend oder vollständig zu entfernen und Rückstand mit einem geeigneten organischen Lösungsmittel zu extrahieren. Dabei wird der Fachmann die Lösungsmittel so auswählen, dass das gewünschte Produkt in ihnen löslich ist, nicht jedoch die bei der Neutralisation anfallenden Salze. Typische organische Lösungsmittel zur Extraktion sind C₂-C₆-Alkohole, wie Ethanol, n-Propanol, iso-Propanol, n-Butanol, Isobutanol, Amylalkohol und Isoamylalkohol, aromatische Kohlenwasserstoffe wie Toluol, Ethylbenzol und xylole. Nach Einengen des Extraktes zur Trockne fällt dann die Zielverbindung IV an und kann in üblicher Weise weiter gereinigt und aufgearbeitet werden.

Ebenso kann man das wässrige Reaktionsmedium der 2. Reaktionsstufe, vorzugsweise nach Neutralisation, extraktiv mit einem polaren, mit Wasser nicht oder nur eingeschränkt mischbaren Lösungsmittel aufarbeiten, beispielsweise durch Extraktion mit einem C₄-C₆-Alkohol wie n-Butanol, Isobutanol, Amylalkohol oder Isoamylalkohol, oder mit einem der vorgenannten aromatischen Kohlenwasserstoffe. Die Extraktion kann portionsweise oder kontinuierlich durchgeführt werden.

Zur Erläuterung des erfindungsgemäßen Verfahrens wird im Folgenden eine typische Verfahrensvorschrift für die Umsetzung der Verbindungen II zu den 2-Pyrazolin-5-onen beschrieben:

Die Verbindungen V werden in einer wässrigen Lösung des Alkalihydroxid gelöst. Die Konzentration der Lösung liegt in der Regel im Bereich von 10 bis 50 Gew.-% und ist so bemessen, dass pro Mol Verbindung V 5 bis 12 Mol Alkalihydroxid vorliegt. Diese Lösung wird in einem Autoklaven auf eine Temperatur im Bereich von 150 bis 180 °C erhitzt, wobei sich ein Druck im Bereich von 5 bis 7 bar aufbaut. Die Reaktionstemperatur wird für 2 bis 10 Stunden beibehalten. Nach Abkühlen auf Raumtemperatur und Entspannen auf Normaldruck gibt man eine zur Einstellung des pH-Werts ausreichende Menge an Mineralsäure zu. Der pH-Wert liegt vorzugsweise im Bereich von 0 bis 6 und insbesondere im Bereich von 1 bis 3. Hierbei tritt eine spontane CO₂-Entwickluug auf. Anschließend neutralisiert man mit einer Base auf pH 6 bis 7. Man dampft die Reaktionsmischung im Vakuum zur Trockne ein und extrahiert den festen Rückstand, beispielsweise in einer Soxhlet-Apparatur mit einem geeignete Lösungsmittel. Nach Verdampfen des Lösungsmittels erhält man das N-substituierte 2-Pyrazolin-5-on der Formel IV in hoher Ausbeute und Reinheit. Anstelle durch Eindampfen/Extrahieren kann man die Verbindung IV aus der wässrigen Reaktionsmischung nach Neutralisation auf pH 6 bis 7 auch durch Extraktion mit einem geeigneten Lösungsmittel, z. B. Isobutanol oder Toluol, isolieren.

Zur Erläuterung des erfindungsgemäßen Verfahrens wird nachfolgend eine typische Verfahrensvorschrift für die Umsetzung der Verbindungen der allgemeinen Formel II zu den N substituierten 5-Chlor-4-methylpyrazolen der allgemeinen Formel I, deren anschließende Oxidation zu einer Verbindung der allgemeinen Formel V sowie die Überführung der Verbindungen V in die N-substituierten 2-Pyrazolin-5-one der allgemeinen Formel IV beschrieben. Diese Beispiele dienen nur der Erläuterung und sind nicht einschränkend zu verstehen.

### Beispiel 1 (Chlorierung von 1,4-Dimethylpyrazol)

In eine Lösung von 192 g (2,0 mol) 1,4-Dimethylpyrazol und 800 g 1,2-Dichlorethan wurden innerhalb von 2 h 190 g (2,67 mol) Chlor eingegast. Die Temperatur stieg auf 60 °C an und ließ sich durch Kühlen mit Eis bei 60 °C halten. Unter Kühlung neutralisierte man die erhaltene Reaktionslösung bei 25 °C mit 650 g (2,43 mol) 15%iger wässriger Natronlauge. Nach Phasentrennung ergab die Destillation der organischen Phase 170,1 g (1,3 mol) 5-Chlor-1,4-dimethylpyrazol Kp₁₂₀: 105 °C mit einem Gehalt von 99,7 % (GC) und 99,3 g (0,6 mol) 3,5-Dichlor-1,4-dimethylpyrazol Kp₁₅: 85 °C mit einem Gehalt von 99,5 % (GC). Die Ausbeuten von 5-Chlor-1,4-dimethylpyrazol und 3,5-Dichlor-1,4-dimethylpyrazol ergaben eine Gesamtausbeute von 95 % bezogen auf das eingesetzte 1,4-Dimethylpyrazol.

### Beispiel 2 (Dehalogenierung von 3,5-Dichlor-1,4-dimethylpyrazol in Eisessig)

12,5 g (0,075 mol) 3,5-Dichlor-1,4-dimethylpyrazol mit einer Reinheit von 99,5 %, 150 g Essigsäure 100%ig, 12,3 g (0,15 mol) Natriumacetat und 6,3 g Pd/C 10%ig Katalysator wurden in einem 350 mL Rührautoklav auf 60 °C erwärmt. Bei dieser Temperatur presste man 30 bar Wasserstoff auf. Die Reaktion setzte sofort ein und nach ca. 3 h war die Wasserstoffaufnahme beendet. Man ließ den Autoklaven auf 25 °C abkühlen, entspannte ihn und filtrierte den Katalysator und das entstandenen Natriumchlorid ab. Die Destillation des Filtrats ergab 6,86 g 1,4-Dimethylpyrazol Kp: 151 °C mit einem Gehalt von 99,7 % (GC). Das entspricht einer Ausbeute von 95 % der Theorie.

### Beispiel 3 (Dehalogenierung von 3,5-Dichlor-1,4-dimethylpyrazol in 1,4-Dimethylpyrazol in Gegenwart von Natriumacetat)

16,6 g (0,1 mol) 3,5-Dichlor-1,4-dimethylpyrazol mit einer Reinheit von 99,5 %, 50 g 1,4-Dimethylpyrazol 99,8%ig, 16,4 g (0,2 mol) Natriumacetat und 6,4 g Pd/C 30%ig Katalysator wurden in einem 350 mL Rührautoklav auf 80 °C erwärmt. Bei dieser Temperatur presste man 40 bar Wasserstoff auf. Nach ca. 6 h war die Wasserstoffaufnahme beendet. Man ließ den Autoklaven auf 25 °C abkühlen, entspannte ihn und filtrierte den Katalysator und das entstandene Natriumchlorid ab. Die Destillation des Filtrats ergab 59 g 1,4-Dimethylpyrazol Kp: 151 °C mit einem Gehalt von 99,8 %. Abzüglich der als Lösungsmittel eingesetzten 50 g 1,4-Dimethylpyrazol entspricht dies einer Ausbeute von 93,6 % der Theorie.

### Beispiel 4 (Dehalogenierung von 3,5-Dichlor-1,4-dimethylpyrazol in 1,4-Dimethylpyrazol in Gegenwart von Natronlauge)

16,6 g (0,1 mol) 3,5-Dichlor-1,4-dimethylpyrazol mit einer Reinheit von 99,5 %, 50 g 1,4-Dimethylpyrazol 99,8%ig, 16,0 g (0,2 mol) 50 gew.-%ige. Natronlauge und 6,4 g Pd/C 30%ig Katalysator wurden in einem 350 mL Rührautoklav auf 80 °C erwärmt. Bei dieser Temperatur presste man 40 bar Wasserstoff auf. Nach ca. 6 h war die Wasserstoffaufnahme beendet. Man ließ den Autoklaven auf 25 °C abkühlen, entspannte ihn und filtrierte den Katalysator und das entstandene Natriumchlorid ab. Die Destillation des Filtrats ergab 58,7 g 1,4-Dimethylpyrazol Kp: 151 °C mit einem Gehalt von 99,8 %. Abzüglich der als Lösungsmittel eingesetzten 50 g 1,4-Dimethylpyrazol entspricht dies einer Ausbeute von 90,0 % der Theorie.

### Beispiel 5 (Dehalogenierung von 3,5-Dichlor-1,4-dimethylpyrazol in 1,4-Dimethylpyrazol in Gegenwart von Calciumhydroxid)

16,6 g (0,1 mol) 3,5-Dichlor-1,4-dimethylpyrazol mit einer Reinheit von 99,5 %, 50 g 1,4-Dimethylpyrazol 99,8%ig, 7,4 g (0,1 mol) Calciumhydroxid, 8 ml Wasser und 6,4 g Pd/C 30%ig Katalysator wurden in einem 350 mL Rührautoklav auf 80°C erwärmt. Bei dieser Temperatur presste man 40 bar Wasserstoff auf. Nach ca. 6 h war die Wasserstoffaufnahme beendet. Man ließ den Autoklaven auf 25 °C abkühlen, entspannte ihn und filtrierte den Katalysator und das entstandene Calciumchlorid ab. Die Destillation des Filtrats ergab 58,2 g 1,4-Dimethylpyrazol Kp: 151 °C mit einem Gehalt von 99,7 %. Abzüglich der als Lösungsmittel eingesetzten 50 g 1,4-Dimethylpyrazol entspricht dies einer Ausbeute von 84,6 % der Theorie.

### Beispiel 6 (Dehalogenierung von 3,5-Dichlor-1,4-dimethylpyrazol in 1,4-Dimethylpyrazol in Gegenwart von Calciumoxid)

16,6 g (0,1 mol) 3,5-Dichlor-1,4-dimethylpyrazol mit einer Reinheit von 99,5 %, 50 g 1,4-Dimethylpyrazol 99,8%ig, 5,6 g (0,1 mol) Calciumoxid, 8 ml Wasser und 6,4 g Pd/C 30%ig Katalysator wurden in einem 350 mL Rührautoklav auf 80 °C erwärmt. Bei dieser Temperatur presste man 40 bar Wasserstoff auf. Nach ca. 6 h war die Wasserstoffaufnahme beendet. Man ließ den Autoklaven auf 25 °C abkühlen, entspannte ihn und filtrierte den Katalysator und das entstandene Calciumchlorid ab. Die Destillation des Filtrats ergab 57,6 g 1,4-Dimethylpyrazol Kp: 151 °c mit einem Gehalt von 99,7 %. Abzüglich der als Lösungsmittel eingesetzten 50 g 1,4-Dimethylpyrazol entspricht dies einer Ausbeute von 78,4 % der Theorie.

### Beispiel 7 (Chlorierung von 1-Ethyl-4-methylpyrazol)

In eine Lösung von 165 g (1,5 mol) 1-Ethyl-4-methylpyrazol und 625 g 1,2-Dichlorethan wurden innerhalb von 2 h 167,7 g (2,36 mol) Chlor eingegast. Die Temperatur stieg auf 60 °C an und ließ sich durch Kühlen mit Eis bei 60 °C halten. Unter Kühlung neutralisierte man die erhaltene Reaktionslösung bei 25 °C mit 533,9 g (2,0 mol) 15%iger wässriger Natronlauge. Nach Phasentrennung ergab die Destillation der organischen Phase 122,1 g (0,843 mol) 5-Chlor-1-ethyl-4-methylpyrazol Kp₂₀₀: 118 °C mit einem Gehalt von 99,8 % (GC) und 109,1 g (0,61 mol) 3,5-Dichlor-1-ethyl-4-methylpyrazol Kp₂₀₀: 154 °C mit einem Gehalt von 99,6 %. Dies entspricht einer Ausbeute von 96,6 % der Theorie bezogen auf das eingesetzte 1-Ethyl-4-methylpyrazol.

### Beispiel 8 (Dehalogenierung von 3,5-Dichlor-1-ethyl-4-methylpyrazol in Eisessig)

18,0 g (0,1 mol) 3,5-Dichlor-1-ethyl-4-methylpyrazol mit einer Reinheit von 99,6 %, 100 g Essigsäure 100%ig, 16,4 g (0,2 mol) Natriumacetat und 8,4 g Pd/C 10%ig Katalysator wurden in einem 350 mL Rührautoklav auf 80 °C erwärmt. Bei dieser Temperatur presste man 20 bar Wasserstoff auf. Die Reaktion setzte sofort ein und nach ca. 2 h war die Wasserstoffaufnahme beendet. Man ließ den Autoklaven auf 25 °C abkühlen, entspannte ihn und filtrierte den Katalysator und das entstandene Natriumchlorid ab. Die Destillation des Filtrats ergab 10,3 g 1-Ethyl-4-methylpyrazol Kp: 158 °C mit einem Gehalt von 99,6 % (GC). Das entspricht einer Ausbeute von 93,4 % der Theorie.

### Beispiel 9 (Dehalogenierung von 3,5-Dichlor-1-ethyl-4-methylpyrazol in 1-Ethyl-4-methylpyrazol)

18,0 g (0,1 mol) 3,5-Dichlor-1-ethyl-4-methylpyrazol mit einer Reinheit von 99,6 %, 50 g 1-Ethyl-4-methylpyrazol mit einer Reinheit von 99,8 %, 16,4 g (0,2 mol) Natriumacetat und 6,4 g Pd/C 30%ig Katalysator wurden in einem 350 mL Rührautoklav auf 80 °C erwärmt. Bei dieser Temperatur presste man 30 bar Wasserstoff auf. Nach ca. 4 h war die Wasserstoffaufnahme beendet. Man ließ den Autoklaven auf 25 °C abkühlen, entspannte ihn und filtrierte den Katalysator und das entstandene Natriumchlorid ab. Die Destillation des Filtrats ergab 60,4 g 1-Ethyl-4-methylpyrazol Kp: 158 °C mit einem Gehalt von 99,7 %, abzüglich der eingesetzten 50 g 1-Ethyl-4-methylpyrazol entspricht das einer Ausbeute von 93,6 % der Theorie.

### Beispiel 10 (Oxidation von 5-Chlor-1,4-dimethylpyrazol)

43,1 g (0,33 mol) 5-Chlor-1,4-dimethylpyrazol, 2,5 g (0,01 mol) Kobalt-(II)-acetat Tetrahydrat, 0,66 g (2,68 mmol) Mangan-(II)-acetat Tetrahydrat, 2,0 g (19,4 mmol) Natriumbromid und 180 g (3,0 mol) Essigsäure 100 %ig wurden in einem 350 mL Rührautoklav auf 130 °C erwärmt. Bei dieser Temperatur wurden 20 bar Sauerstoff aufgepresst. Die Reaktion setzte sofort ein. Sauerstoff wurde mehrmals nachgepresst. Nach etwa 5 Stunden wurde kein Sauerstoff mehr verbraucht. Man kühlte auf Raumtemperatur ab und entspannte den Autoklaven. Das erhaltene Reaktionsgemisch wurde am Rotationsverdampfer eingeengt. Der erhaltene Rückstand wurde aus 300 mL 20 gew.%iger, wässriger Essigsäure umkristallisiert. Nach dem Trocknen erhielt man 44,1 g 5-Chlor-l-methyl-4-pyrazolcarbonsäure mit einem Gehalt von 99,2 % (HPLC). Das entspricht einer Ausbeute von 82,6 % der Theorie. Der Schmelzpunkt lag bei 197 °C.

### Beispiel 11 (Oxidation von 5-Chlor-1-ethyl-4-methylpyrazol)

Die Ansatzgröße und Durchführung entsprach dem Beispiel 7. Man setzte 47,7 g (0,33 mol) 5-Chlor-1-ethyl-4-methylpyrazol ein. Nach dem Trocknen erhielt man 46,0 g 5-Chlor-1-ethyl-4-pyrazol-carbonsäure mit einem Gehalt von 99,5 % (HPLC). Dies entspricht einer Ausbeute von 79,5 % der Theorie. Der Schmelzpunkt betrug 208 °C.

### Beispiel 12 (Oxidation von 5-Chlor-1,4-dimethylpyrazol)

26,1 g (0,2 mol) 5-Chlor-1,4-dimethylpyrazol, 6,6 g (0,026 mol) Cobalt-(II)-acetat Tetrahydrat, 6,0 g (0,035 mol) Bromwasserstoffsäure 47 %ig, 2,0 g (0,017 mol) Wasserstoffperoxid 30 %ig und 240 g (4,0 mol) Essigsäure 100 %ig wurden in einem 350 mL Rührautoklav auf 90 °C erhitzt. Bei dieser Temperatur presste man 30 bar Sauerstoff auf. Die Reaktion setzte sofort ein. Man presste mehrmals den Sauerstoff nach. Nach etwa 6 Stunden war der Sauerstoffverbrauch beendet. Man kühlte auf Raumtemperatur ab und entspannte den Autoklaven. Das erhaltene Reaktionsgemisch wurde am Rotationsverdampfer eingeengt. Die Umkristallisation aus 150 mL 20 gew.%iger, wässriger Essigsäure ergab nach dem Trocknen 26,5 g 5-Chlor-1-methyl-4-pyrazolcarbonsäure mit einem Gehalt von 98,7 % (HPLC). Das entspricht einer Ausbeute von 81,5 % der Theorie. Der Schmelzpunkt betrug 195 °C.

### Beispiel 13 (Herstellung von 1-Methyl-2-pyrazolin-5-on)

In einem 250 ml Autoklaven löste man 10 g (0,0623 mol) 5-Chlor-l-methyl-4-pyrazolcarbonsäure in 100 g 25 gew.-%iger Natronlauge (= 0,623 mol). Die Lösung erhitzte man 6 h auf 175 °C. Hierbei stieg der Druck auf 6 bar an. Nach dem Abkühlen entspannte man auf Normaldruck. Die Reaktionsmischung wurde anschließend mit 60 gew.-%iger Schwefelsäure auf pH 1,5 eingestellt. Hierbei trat eine CO₂-Entwicklung auf. Nach einigen Minuten stellte man mit 25 gew.-%iger Natronlauge einen pH-Wert von 6,5 ein und engte die erhaltene Lösung im Vakuum zur Trockne ein. Der feste Rückstand wurde in eine Soxhlet-Apparatur überführt und mit Ethanol kontinuierlich extrahiert. Nach Abdestillieren des Ethanols im Vakuum erhielt man 5,7 g der Zielverbindung mit einem Gehalt von 98,9 % (bestimmt mittels Gaschromatographie). Der Schmelzpunkt lag bei 113 °C. Dies entspricht einer Ausbeute von 92,3 % der Theorie. Das Produkt wurde durch Mischschmelzpunkt mit einer authentischen Probe identifiziert.

### Beispiel 14 (Herstellung von 1-Ethyl-2-pyrazolin-5-on = 5-Hydroxy-l-ethylpyrazol)

4 g 5-Chlor-1-ethyl-4-pyrazolcarbonsäure wurden in 40 g 25 gew.-%iger Natronlauge gelöst und analog der in Beispiel 1 beschriebenen Vorgehensweise umgesetzt. Die Reaktionstemperatur der ersten Reaktionsstufe lag bei 170 °C, der Reaktionsdruck bei 7,5 bar. Die Reaktionsdauer betrug 8 h. Bei Aufarbeitung in der für Beispiel 1 beschriebenen weise erhielt man 2,3 g der Zielverbindung mit einem Gehalt von 99,7 % (bestimmt mittels Gaschromatographie). Dies entspricht einer Ausbeute von 89,4 % der Theorie. Der Schmelzpunkt lag bei 88 °C. Das Produkt wurde durch Mischschmelzpunkt mit einer authentischen Probe identifiziert.

### Beispiel 15 (Herstellung von 1-Methyl-2-pyrazolin-5-on, Aufarbeitung durch flüssig-flüssig-Extraktion)

10 g 5-Chlor-1-methylpyrazol-4-carbonsäure wurden wie in Beispiel 1 zunächst mit 100 g 25 gew.-%iger Natronlauge und anschließend unter sauren Bedingungen umgesetzt. Nach Neutralisation der sauren Reaktionsmischung mit 25 gew.-%iger Natronlauge auf pH 6,5 wurde die Reaktionsmischung in einen flüssig-flüssig-Extraktor überführt und mit Isobutanol bei Siedetemperatur des Lösungsmittels extrahiert. Nach Isolierung der organischen Phase und Abdestillieren des Isobutanols verblieben 5,8 g 1-Methyl-2-pyrazolinon zurück (Reinheit laut GC: 98,1 %). Der Schmelzpunkt lag bei 112 °C. Die Ausbeute betrug 92,5 % der Theorie.

## Patentansprüche

1. Verfahren zur Herstellung von 1-substituierten S-Chlor-4-methylpyrazolen der Formel I 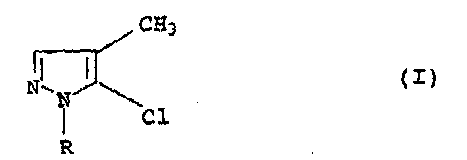 worin
R für C₁-C₈-Alkyl oder C₅-C₁₀-Cycloalkyl steht, die gegebenenfalls einen oder mehrere Substituenten aufweisen,
durch Umsetzung eines 4-Methylpyrazols der allgemeinen Formel II. 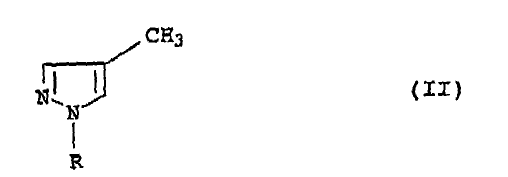 worin
R die oben genannten Bedeutungen aufweist, mit Chlor, wobei man ein Gemisch aus Verbindung I und einer 1-substituierten 3,5-Dichlor-4-methylpyrazol-Verbindung III erhält, 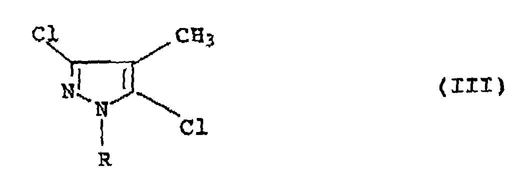 worin
R die zuvor genannten Bedeutungen hat, **dadurch gekennzeichnet, dass** man die Verbindung III von der Verbindung I abtrennt, die Verbindung III zu der Verbindung II durch katalytische Hydrogenolyse enthalogeniert und diese in die Umsetzung von II mit Chlor zurückführt.

2. Verfahren nach Anspruch 1, **dadurch gekennzeichnet, dass** man die Enthalogenierung mit Wasserstoff in Gegenwart von Palladium als Katalysator durchführt.

3. Verfahren nach Anspruch 2, **dadurch gekennzeichnet, dass** der Katalysator auf Aktivkohle geträgertes Palladium ist.

4. Verfahren nach einem der vorhergehenden Ansprüche, **dadurch gekennzeichnet, dass** man die Verbindung III vor der Enthalbgenierung mit der zu chlorierenden Verbindung II vereinigt.

5. Verfahren zur Herstellung von 1-substituierten Pyrazolonen der Formel IV, 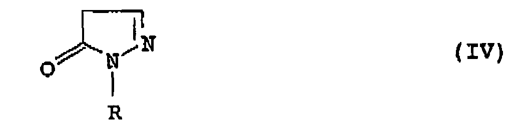 **dadurch gekennzeichnet, dass** man in einem ersten Reaktionsschritt ein 1-substituiertes 5-Chlor-4-methylpyrazol der allgemeinen Formel I nach einem Verfahren gemäß einem der vorhergehenden Ansprüche herstellt, anschließend die 4-Methylgruppe in der Verbindung I zu einer carboxylgruppe oxidiert, das hierbei erhaltene 4-Carboxy-5-chlorpyrazol der Formel V, 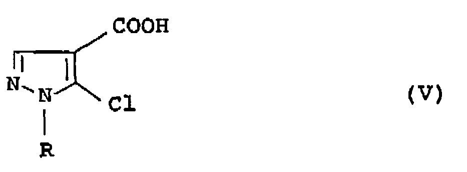 worin
R die im Anspruch 1 genannten Bedeutungen aufweist, in einem wässrigen Reaktionsmedium bei erhöhter Temperatur mit einem molaren Überschuss Alkalihydroxid umsetzt und anschließend durch Zugabe einer Säure einen pH-wert ≤ 6 im wässrigen Reaktionsmedium einstellt.

6. Verfahren nach Anspruch 5, **dadurch gekennzeichnet, dass** man die Verbindung der allgemeinen Formel V mit wenigstens 3 Mol Alkalihydroxid, bezogen auf 1 Mol der Verbindung V umsetzt.

7. Verfahren nach einem der vorhergehenden Ansprüche 5 und 6, **dadurch gekennzeichnet, dass** man die Umsetzung mit wässrigem Alkalihydroxid bei einer Temperatur oberhalb 90 °C durchführt.

8. Verfahren nach einem der vorhergehenden Ansprüche 5 bis 7, **dadurch gekennzeichnet, dass** die Zugabe der Säure bei einer Temperatur im Bereich von 0 bis 100 °C erfolgt.

## Claims

1. A process for preparing 1-substituted 5-chloro-4-methylpyrazoles of the formula I 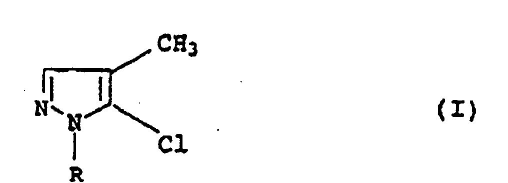 in which
R is C₁-C₈-alkyl or C₅-C₁₀-cycloalkyl, each of which optionally has one or more substituents,
by reacting a 4-methylpyrazole of the formula II 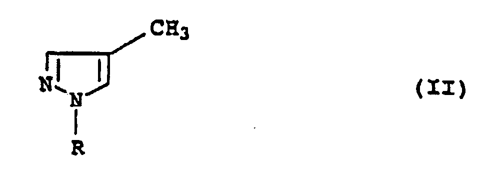 in which
R has the abovementioned meanings, with chlorine, resulting in a mixture of compound I and a 1-substituted 3,5-dichloro-4-methylpyrazole compound III 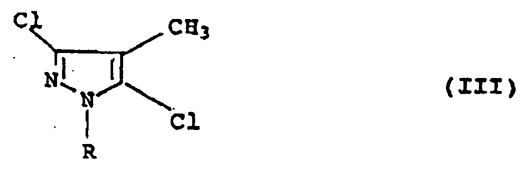 in which
R has the aforementioned meanings, wherein the compound III is separated from the compound I, the compound III is dehalogenated by catalytic hydrogenolysis to give the compound II, and the latter is returned to the reaction of II with chlorine.

2. The process according to claim 1, wherein the dehalogenation is carried out with hydrogen in the presence of palladium as catalyst.

3. The process according to claim 2, wherein the catalyst is palladium supported on activated carbon.

4. The process according to any of the preceding claims, wherein compound III I is combined before the dehalogenation with the compound II to be chlorinated.

5. A process for preparing 1-substituted pyrazolones of the formula IV 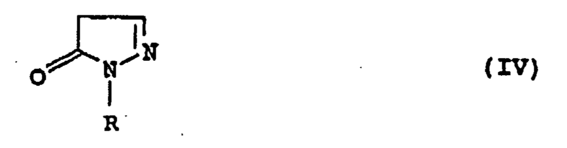 which comprises preparing in a first reaction step a 1-substituted 5-chloro-4-methylpyrazole of the formula I by a process according to any of the preceding claims, subsequently oxidizing the 4-methyl group in the compound I to a carboxyl group, reacting the 4-carboxy-5-chloropyrazole of the formula V obtained in this way 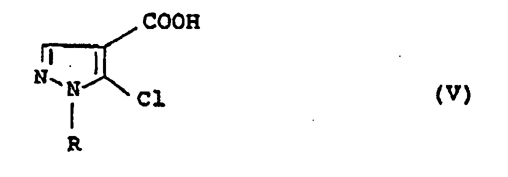 in which
R has the meanings stated in claim 1, with a molar excess of alkali metal hydroxide in an aqueous reaction medium at elevated temperature, and subsequently adjusting a pH of ≤ 6 in the aqueous reaction medium by adding an acid.

6. The process according to claim 5, wherein the compound of the formula V is reacted with at least 3 mol of alkali metal hydroxide based on 1 mol of the compound V.

7. The process according to either of preceding claims 5 and 6, wherein the reaction with aqueous alkali metal hydroxide is carried out at a temperature above 90°C.

8. The process according to any of preceding claims 5 to 7, wherein the acid is added at a temperature in the range from 0 to 100°C.

## Revendications

1. Procédé de préparation de 5-chloro-4-méthylpyrazoles 1-substitués de formule I 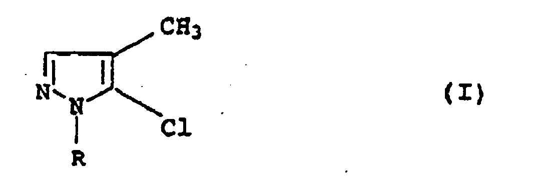 dans laquelle
R représente un groupe alkyle en C₁-C₈ ou un groupe cycloalkyle en C₅-C₁₀, qui renferment éventuellement un ou plusieurs substituants,
par la réaction d'un 4-méthylpyrazole de formule générale II, 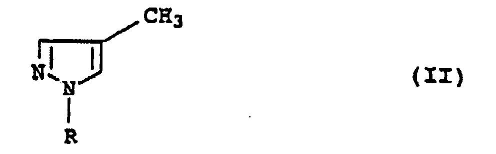 dans laquelle
R présente les significations mentionnées ci-dessus, avec du chlore, en donnant lieu à un mélange du composé I et d'un composé 3,5-dichloro-4-méthylpyrazole 1-substitué III, 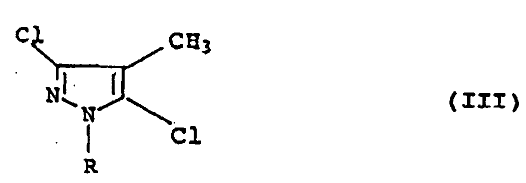 dans lequel
R présente les significations mentionnées précédemment, **caractérisé en ce que** le composé III est séparé du composé I, le composé III est déshalogéné par hydrogénolyse catalytique pour donner lieu au composé II et celui-ci est renvoyé à la réaction de II avec du chlore.

2. Procédé selon la revendication 1, **caractérisé en ce que** la déshalogénation est réalisée avec de l'hydrogène en présence de palladium en tant que catalyseur.

3. Procédé selon la revendication 2, **caractérisé en ce que** le catalyseur est du palladium supporté sur du charbon actif.

4. Procédé selon l'une quelconque des revendications précédentes, **caractérisé en ce que** le composé III est combiné avant la déshalogénation avec le composé II destiné à être chloré.

5. Procédé de préparation de pyrazolones 1-substitués de formule IV, 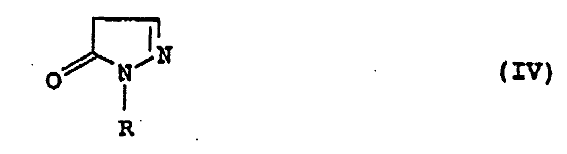 **caractérisé en ce que**, dans une première étape de réaction, un 5-chloro-4-méthylpyrazole 1-substitué de formule générale I est préparé conformément à un procédé selon l'une des revendications précédentes, puis le groupe 4-méthyle dans le composé I est oxydé pour donner lieu à un groupe carboxyle, le 4-carboxy-5-chloropyrazole ainsi obtenu de formule V, 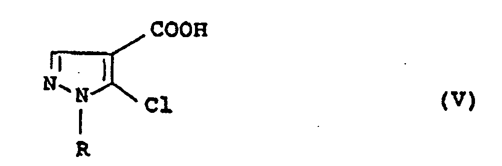 dans laquelle
R présente les significations mentionnées dans la revendication 1, est mis à réagir avec un excès molaire d'hydroxyde de métal alcalin dans un milieu réactionnel aqueux à une température accrue, puis un pH ≤ 6 est établi dans le milieu réactionnel aqueux par l'addition d'un acide.

6. Procédé selon la revendication 5, **caractérisé en ce que** le composé de formule générale V est mis à réagir avec au moins 3 mol d'hydroxyde de métal alcalin, par rapport à 1 mol du composé V.

7. Procédé selon l'une quelconque des revendications 5 et 6 précédentes, **caractérisé en ce que** la réaction avec l'hydroxyde de métal alcalin aqueux est réalisée à une température supérieure à 90°C.

8. Procédé selon l'une quelconque des revendications 5 à 7 précédentes, **caractérisé en ce que** l'addition de l'acide est réalisée à une température dans la plage de 0 à 100°C.
